# EUROPEAN PATENT APPLICATION

(11) **EP 2 829 286 A1**
(43) Date of publication of application: **28.01.2015**
(21) Application number: 14178353.0
(22) Date of filing: 24.07.2014
(51) Int. Cl.: A61L 17/10

(54) **Hybrid suture with monofilament and braided construction**

(30) Priority: 24.07.2013 US 201361857818 P
(71) Applicant: Arthrex, Inc., Naples, FL 34108-1945 (US)
(72) Inventor: Dreyfuss, Peter J, Naples, FL Florida 34108 (US); Dooney, JR., Thomas, Naples, FL Florida 34109 (US)
(74) Representative: Ehrner & Delmar Patentbyrå AB

(57) **Abstract**

A hybrid monofilament/braided surgical suture that combines a monofilament with a braided construction. The hybrid surgical suture may be formed by braiding yarns of high tenacity fibers tightly over a core in the form of a monofilament yarn. The monofilament may be any type of material, for example, a nylon, silk, polyester, polyethylene or polypropylene filament, among many others. The stiffness properties of the hybrid surgical suture allow the suture to be pushed through small and very small diameter tubes and cannulations (such as Lasso instruments, for example) yet fix securely with existing fixation devices (such as knotless suture anchors).

## Description

### FIELD OF THE INVENTION

The present invention relates to surgical suture materials and, in particular, to improved braided monofilament sutures.

### BACKGROUND OF THE INVENTION

Sutures have been known throughout the history of surgery. Recently, the variety of materials used to close wounds and attach soft tissue to bone (or soft tissue to soft tissue) has included a wide selection of synthetic compositions such as ultrahigh molecular weight polyethylene (UHMWPE), or the FiberWire® suture described in U.S. Patent No. 6,716,234, or the FiberTape® suture tape described in U.S. Patent No. 7,892,256.

There is a need for an improved suture with could fold easily to provide a construct with a reduced diameter for tissue passing and instrument passing. Also needed are improved sutures with handling characteristics of the monofilaments (i.e., capable of passing through small cannulations of suture passing instruments) but also capable of fixing well in anchors that rely on friction for holding suture without knots. Also needed are methods of suture manipulation with decreased suture tear, as well as methods of maximizing benefits of tissue cut-through resistance with minimal and efficient use of material.

### SUMMARY OF THE INVENTION

The present invention provides a surgical suture that combines a monofilament with a braided construction. The surgical suture is a hybrid monofilament/braided suture. The hybrid suture passes easily through any suture passing instrument (even through ones having a very small diameter) while fixes securely with hard fixation devices such as knotless PushLock® anchors to increase tissue fixation and strength.

The hybrid surgical suture may be formed by braiding yarns of high tenacity fibers tightly over a core in the form of a monofilament yarn. The monofilament may be any type of material, for example, a nylon, silk, polyester, polyethylene or polypropylene filament, among many others. The stiffness properties of the hybrid surgical suture of the present invention allows the suture to be pushed through small and very small diameter tubes and cannulations (such as Lasso instruments, for example) yet fix securely with existing fixation devices (such as knotless suture anchors like PushLock® anchors).

The hybrid surgical suture may be formed by inserting a monofilament yarn (monofilament core) into a braid such as a FiberWire® CL braid (a coreless braid with UHMWPE). The resulting hybrid surgical suture is not a coreless construct anymore. The monofilament may be any type of material, for example, a yarn of nylon, polyester, polyethylene, silk, etc. The monofilament yarn is fed into the core as the jacket is being braided.

Other features and advantages of the present invention will become apparent from the following description of the invention, which refers to the accompanying drawings.

### BRIEF DESCRIPTION OF THE DRAWINGS

FIG. 1 illustrates a hybrid monofilament/braided suture according to an exemplary embodiment of the present invention.
FIG. 2 illustrates a partial, cross-sectional view of the hybrid monofilament/braided suture of FIG. 1.
FIGS. 3-8 illustrate another hybrid monofilament/braided suture according to another exemplary embodiment of the present invention.

### DETAILED DESCRIPTION OF THE PREFERRED EMBODIMENTS

In the following detailed description, reference is made to various specific embodiments in which the invention may be practiced. These embodiments are described with sufficient detail to enable those skilled in the art to practice the invention, and it is to be understood that other embodiments may be employed, and that structural and logical changes may be made without departing from the scope of the present invention.

The present invention provides a surgical suture that combines a monofilament with a braided construction. The surgical suture is a hybrid monofilament/braided suture. The surgical suture combines a monofilament with a braided construction. The suture has handling characteristics of a monofilament (i.e., capable of passing through small tortuous cannulations) but also capable of fixing well in anchors that rely on friction for holding suture without knots.

In an exemplary embodiment, the hybrid surgical suture is formed by braiding yarns of high tenacity fibers tightly over a monofilament yarn. The monofilament may be any type of material, for example, a nylon yarn such as a #1 nylon filament, a polyester yarn, a polyethylene yarn, a polypropylene yarn, etc. The stiffness properties of the hybrid surgical suture of the present invention allows the suture to be pushed through small and very small tubes and cannulations (such as suture passing devices like Lasso instruments, for example) yet fix securely with existing fixation devices (such as knotless suture anchors like PushLock® anchors).

In another exemplary embodiment, the hybrid surgical suture is formed by inserting a monofilament yarn into a braid such as a FiberWire® CL braid (a coreless braid with less than 75% UHMWPE). The resulting hybrid surgical suture is not a coreless construct anymore. The monofilament may be any type of material, for example, a yarn of nylon, polyester, polyethylene, polypropylene or any other similar materials. The monofilament yarn is fed into the braided cover (into the core) as the jacket is being braided.

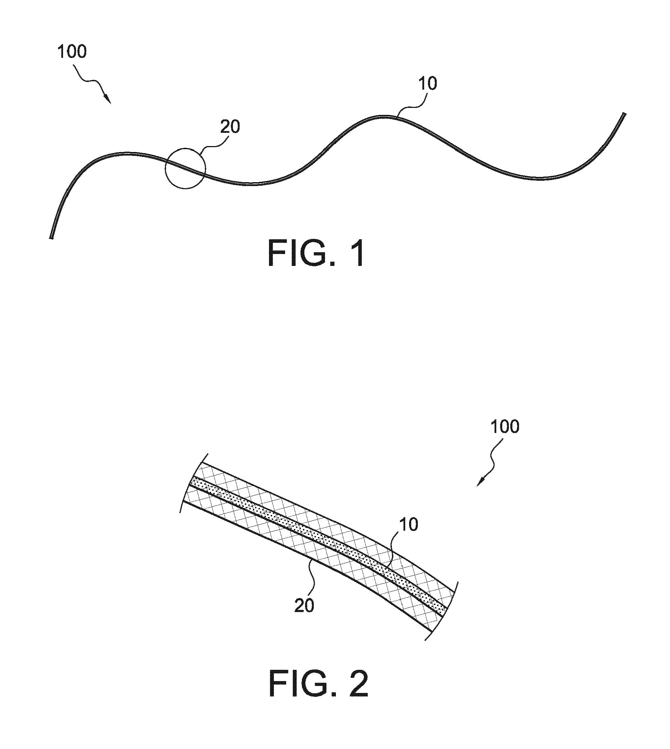

Referring now to the drawings, where like elements are designated by like reference numerals, FIGS. 1 and 2 illustrate an exemplary embodiment of suture 100 of the present invention which is a hybrid monofilament/braided suture comprising a core formed of a monofilament and a jacket (cover) braided over the monofilament.

As shown in FIGS. 1 and 2, suture 100 comprises a section or core 10 substantially formed of a monofilament yarn 10 (monofilament 10 or core 10 or monofilament core 10) and a jacket 20 formed over and in contact with the monofilament 10 during the fabrication process. Monofilament yarn 10 may be formed of any type of material such as, for example, nylon in the form of a #1 nylon filament, or other similar materials such as polyester, polyethylene, or polypropylene, among many others. In an exemplary embodiment only, monofilament 10 is totally covered by cover or jacket 20. The monofilament 10 is preferably formed of nylon yarns or filaments, as nylon is readily available.

In an exemplary embodiment, the jacket 20 is formed essentially of a braid such as a FiberWire® CL braid, which is a coreless braid with ultrahigh molecular weight polyethylene (UHMWPE). As the jacket 20 is being braided, the monofilament 10 is fed into the core so that the UHMWPE yarns are braided over the monofilament 10. The jacket 20 extends along the longitudinal axis of the monofilament 10.

At least one of suture sections 10, 20 (monofilament 10 and braided jacket 20) may be coated (partially or totally) with wax (beeswax, petroleum wax, polyethylene wax, or others), silicone (Dow Coming silicone fluid 202A or others), silicone rubbers (Nusil Med 2245, Nusil Med 2174 with a bonding catalyst, or others) PTFE (Teflon, Hostaflon, or others), PBA (polybutylate acid), ethyl cellulose (Filodel) or other coatings, to improve lubricity of the final hybrid suture, knot security, pliability, handleability or abrasion resistance, for example.

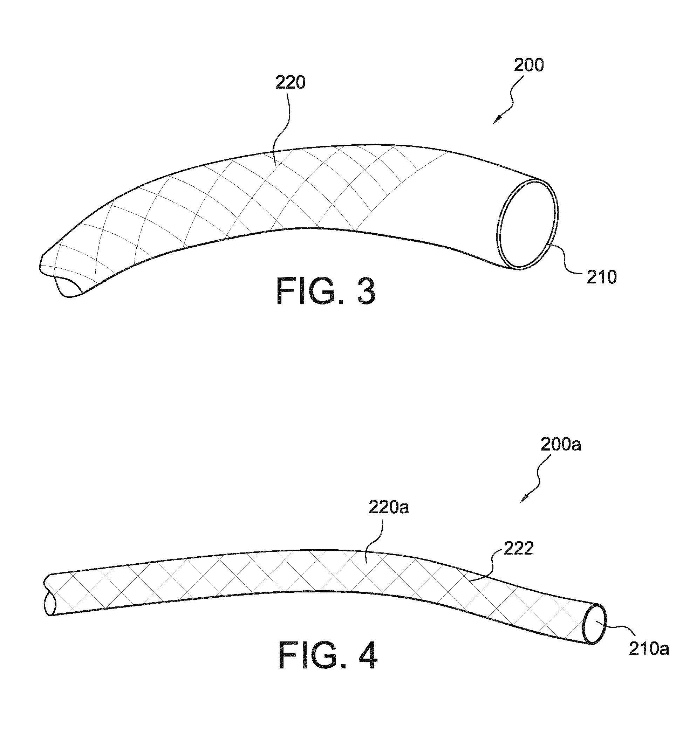

FIGS. 3-8 illustrate additional embodiments of hybrid suture 200, 200a of the present invention, which is another suture with a monofilament core and a braided high strength jacket. Current high strength sutures are either all braided or braided over a soft core. These sutures have high tensile and knot strength but do not have any column/compressive strength. By the present invention, the addition of a monofilament core provides a compressive load property to the suture while maintaining a high tensile strength from the braided jacket. The benefits of this addition are as follows: 1) allows the suture to be pushed through a device instead of being pulled; 2) helps to easily differentiate the suture from other sutures during surgery; 3) helps maintain the perimeter of a pre-made suture geometry like a suture loop; 4) monofilament can be placed in a specific section of the suture where compression is required while the rest of the suture is normal (this would help allow the user to tie knots); and 5) the mono could be part of the braid in a coreless type of suture to help with suture differentiation where compression is not needed.

FIG. 3 illustrates hybrid suture 200 with monofilament core 210 and braided jacket 220. FIG. 4 illustrates hybrid suture 200a with monofilament core 210a and braided jacket 220a; suture 200a includes a braided suture with strand on monofilament 222 within the braid. The addition of a small monofilament to the weave (braid) adds some stiffness to the overall suture while maintaining the same diameter of the suture (but changing the "loose" feel of the suture).

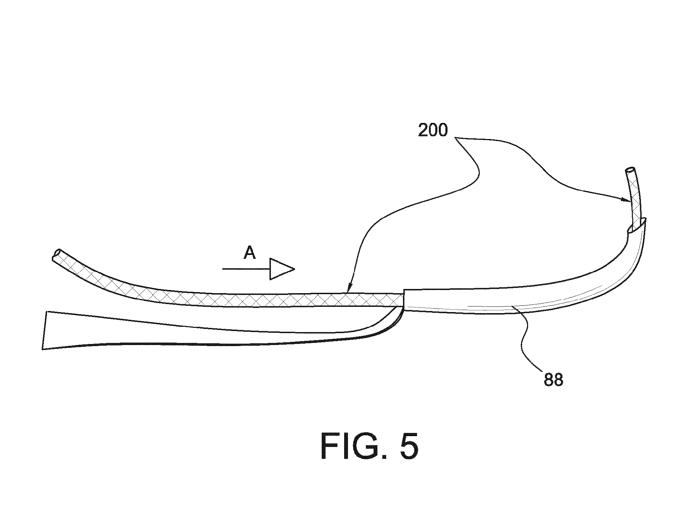

FIG. 5 illustrates exemplary hybrid suture 200 pushed through a surgical device 88 (for example, a cannulated metal suture passer 88) in the direction of arrow A. The braid suture with monofilament core easily passes through the instrument 88.

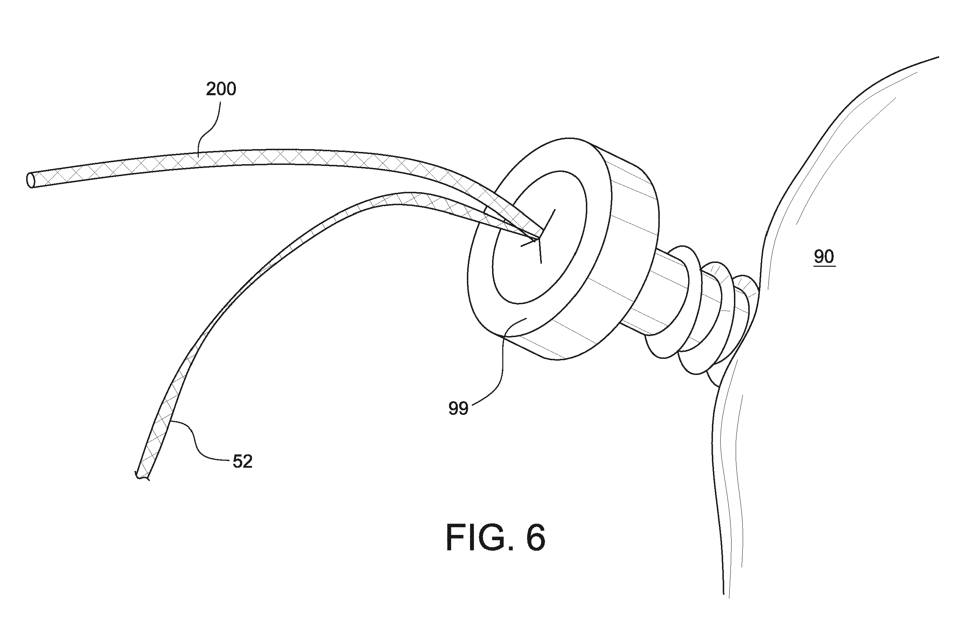

FIG. 6 illustrates braided suture with monofilament core 200 as a stiffer suture in the vicinity of a softer suture 52 (for example, a currently fabricated braided suture), both inserted into a cannula 99, for example, an arthroscopic cannula 99 attached to a part of the human body, for example, humerus 90. Suture 200 is easily differentiated from suture 52 even when they have the same size, i.e., same diameter.

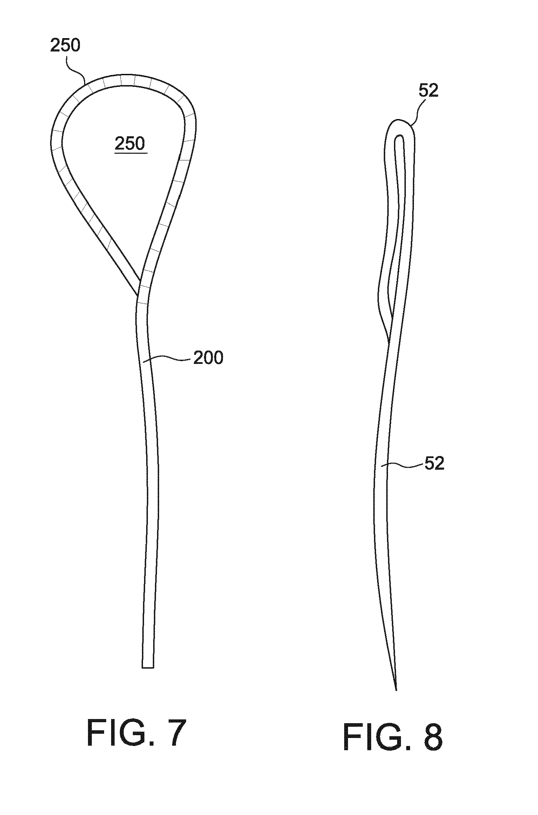

FIG. 7 illustrates how suture 200 keeps loop 250 open when folded, i.e., the monofilament 210 keeps the loop open. The monofilament 210 may be placed only where the loop 250 is forming, i.e., only in a specific section of the suture where compression is required, while the rest of the suture remains normal (i.e., without a monofilament core). This embodiment allows a user to also form one or more knots. If compression is not needed, then the monofilament 210 may be placed within the braid (in a specific or non-specific pattern and at a specific or non-specific region of the braid) to help with suture differentiation. In this embodiment, the braid may be a coreless type of suture.

FIG. 8 illustrates how prior art suture 52 collapses when folded, i.e., the suture could not maintain the loop without the monofilament.

In an exemplary only embodiment, hybrid suture 200 is formed by placing a 3/0 monofilament into the core of a 2/0 FiberLink® (which does not have stiffness). The FiberLink® (and also TigerLink®) is #2 FiberWire® with a closed loop on one end. The FiberLink® is designed to facilitate the application of a cinch stitch by simply passing the suture through tissue and feeding the free end through the loop and tensioning. The cinch stitch can then be locked into position with a knotless anchor like a PushLock® anchor. By the present invention, the insertion of the monofilament opens the link (similar to opening loop 250 as detailed above), adding stiffness to the overall construct while minimally changing the overall diameter of the suture construct.

In yet additional embodiments, and by the present invention, the FiberLink® may be modified to replace at least part of the material forming the FiberLink® (for example, polyester or polyethylene) with a small monofilament. This replacement allow the overall hybrid suture to still maintain the same diameter (for example, 2/0 FiberLink®) and make it feel stiffer. The addition of the monofilament would also help differentiate the suture construct and make it easier to handle the suture (for example, to hold it or grab it) when it is wet and/or alongside of other sutures.

Hybrid suture 100, 200, 200a may have cross-sections of various forms and geometries, including round, oval, rectangular, or flat, among others, or combination of such forms and geometries. The diameter of hybrid suture 100, 200, 200a may be constant or may vary.

At least a part of the fibers of cover 20, 220, 220a (jacket 20, 220, 220a) may contain strands of a high strength suture material, such as Arthrex FiberWire^{®} suture disclosed in U.S. Patent No. 6,716,234, with optional colored strands to assist surgeons in distinguishing between suture lengths with the biological material and suture lengths without the biological material. If desired, the hybrid suture may be provided with tail regions which may have a very fine end that is coated, impregnated, or otherwise stiffened with a material such as plastic, for example.

Cover 20, 220, 220a of hybrid suture 100, 200, 200a may be formed of suture materials, such as PET, UHMWPE, PEEK, silk nylon, and absorbable polymers, among many others. Cover 20, 220, 220a may also contain a bioabsorbable material, such as PLLA or one of the other polylactides, for example, and/or may be formed of twisted fibers having strands of a contrasting color added to the braided threads, to make the suture more visible during surgical procedures. The colored strands, preferably, may be dyed filaments or strands. In exemplary embodiments, cover 20, 220, 220a may be formed of twisted filaments that are then braided directly over the monofilament core 10, 210, 210a. In yet additional embodiments, various sets of yarns (of similar or dissimilar materials) may be weaved, braided, intertwined, or interlinked together by known methods in the art, to form the braided jacket or cover 20, 220, 220a.

The hybrid suture 100, 200, 200a of the present invention has applicability to suture applications that may be employed in surgical procedures such as rotator cuff repair, Achilles tendon repair, patellar tendon repair, ACL/PCL reconstruction, hip and shoulder reconstruction procedures, and applications for suture used in or with suture anchors. In exemplary embodiments only, the suture construct 100, 200, 200a of the present invention may be employed in suture applications that do not involve knot tying, for example, for use with suture anchors (such as PushLock® suture anchor) or for knotless arthroscopic suture repairs (such as knotless single row rotator cuff repair, or SpeedBridge™ repair using no knots and only suture passing steps), among many others. One or more hybrid sutures 100, 200, 200a of the present invention may be provided already pre-loaded on fixation devices, for example, knotless suture anchors such as PushLock® suture anchors.

Although the present invention has been described in connection with preferred embodiments, many modifications and variations will become apparent to those skilled in the art. While preferred embodiments of the invention have been described and illustrated above, it should be understood that these are exemplary of the invention and are not to be considered as limiting.

## Claims

1. A surgical suture, consisting of:
a monofilament core having a longitudinal axis; and
a braided sheath surrounding at least part of the monofilament core and extending along the longitudinal axis of the monofilament core.

2. The surgical suture of claim 1, wherein the monofilament core is formed of nylon, polyester, polyethylene or polypropylene.

3. The surgical suture of claim 1, wherein the monofilament core is a continuous filament of nylon.

4. The surgical suture of claim 1, wherein the monofilament core has a uniform diameter of about 1mm.

5. The surgical suture of claim 1, wherein the braided sheath is a coreless braid formed of twisted yarns of ultrahigh molecular weight polyethylene that are braided together.

6. The surgical suture of claim 1, wherein the suture is formed by inserting the monofilament core into the braided sheath while the braided sheath is being braided.

7. The surgical suture of claim 1, wherein the monofilament core is part of the braided sheath.

8. The surgical suture of claim 1, wherein the braided sheath completely surrounds the monofilament core.

9. The surgical suture of claim 1, wherein the length of the monofilament core is smaller than the length of the braided sheath.

10. The surgical suture of claim 1, wherein the suture has a stiffness that allows the suture to be pushed through a cannulation of a surgical instrument.

11. The surgical suture of claim 1, further comprising a coating provided over the braided sheath.

12. The suture of claim 11, wherein the coating is a silicon or collagen coating.

13. A suture construct, comprising:
a suture strand having a first end which is a free end and a second end which is a closed loop; and
a monofilament core inserted within the closed loop so that the suture strand completely covers the monofilament core.

14. The suture construct of claim 13, wherein the suture strand is a coreless braid formed of yarns of ultrahigh molecular weight polyethylene that are braided together.

15. The suture construct of claim 13, wherein the monofilament core has a diameter of 3mm and the suture strand has a diameter of 2mm.
